(19) Europäisches Patentamt
European Patent Office
Office européen des brevets

(11) Numéro de publication: **0 088 707**
**A1**

(12) **DEMANDE DE BREVET EUROPEEN**

(21) Numéro de dépôt: **83420031.3**

(22) Date de dépôt: **02.03.83**

(51) Int. Cl.³: **C 07 D 471/04**
//
C07D213/12, A61K31/435, (C07D471/04, 221/00, 221/00)

(30) Priorité: **04.03.82 FR 8203798**

(43) Date de publication de la demande: **14.09.83**
**Bulletin 83/37**

(84) Etats contractants désignés: **AT BE CH DE FR GB IT LI LU NL SE**

(71) Demandeur: **RHONE-POULENC S.A., 25, quai Paul Doumer, F-92408 Courbevoie (FR)**

(72) Inventeur: **Erre, Claude-Henry, 11 allée des Cigales, St-Henry F-13016 Marseille (FR)**
Inventeur: **Roussel, Christian, 69, boulevard de la Libération, F-13001 Marseille (FR)**

(74) Mandataire: **Rioufrays, Roger et al, RHONE-POULENC RECHERCHES Centre de Recherches de Saint-Fons Service Brevets B.P. 62, F-69190 Saint-Fons (FR)**

(54) **Nouvelles polyalkylnaphtyridines-2,7 et leur procédé de préparation.**

(57) Nouvelles polyalkylnaphthyridines-2,7 et notamment nouvelles tétraalkyl-1,3,6,8 naphthyridines-2,7 et leur procédé de préparation par polyacylation d'une isooléfine ou d'un précurseur d'isooléfine tel qu'un halogénure tertiaire, un alcool tertiaire, puis traitement du produit de polyacylation par l'ammoniac ou un sel d'ammonium en milieu anhydre.

Les nouvelles polyalkylnaphthyridines peuvent être utilisées comme intermédiaires en synthèse organique.

EP 0 088 707 A1

# NOUVELLES POLYALKYLNAPHTHYRIDINES-2,7 ET LEUR PROCEDE DE PREPARATION

La présente invention a pour objet de nouvelles polyalkylnaphthyridines-2,7 et leur procédé de préparation.

Les composés organiques comportant dans leur structure un reste naphthyridinyle et plus particulièrement un reste naphthyridine-2,7 yle possèdent d'intéressantes propriétés pharmacologiques. Certains d'entre eux peuvent être utilisés comme bactéricides ou fongicides (cf. brevet américain No. 3 928 367), d'autres pour combattre l'hypertension. En dépit de leur intérêt, le développement de ces produits est fortement entravé par les difficultés inhérentes à la synthèse du squelette de la naphthyridine-2,7 :

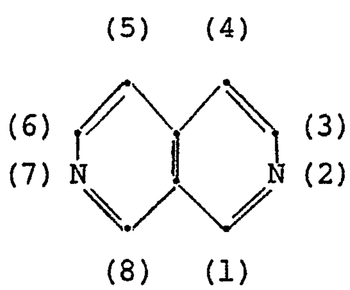

En effet la création de la structure naphthyridine-2,7 yle implique le recours à des procédés complexes et/ou à des matières premières qui nécessitent elles-mêmes la mise en oeuvre de multiples étapes pour leur préparation. Un premier groupe de procédés fait appel à des composés de départ déjà fortement élaborés et qui comportent un noyau pyridyle. C'est ainsi que selon le brevet américain No. 3 928 367 les amino-1 naphthyridine-2,7 sont préparées par condensation d'une cyano-3 alkylpyridine sur un nitrile ou une autre cyano-3 alkyl-4 pyridine dans un solvant aprotique en présence d'une base forte telle qu'un alcoolate alcalin tertiaire (t-butoxyde de sodium par exemple). On a encore fait appel comme produit de départ à l'imide de formule :

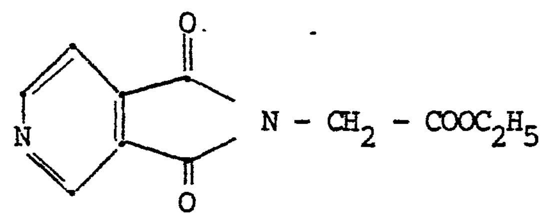

ou à l'acide méthyl-4 nicotinique ou bien encore à la cyano-3 styryl-4 pyridine [cf. W.W. PAUDLER et T.J.KRESS Advances in Heterocyclic Chemistry Volume 11 (1970) pages 155 à 158]. Les procédés qui recourent à ces produits mettent en oeuvre plusieurs étapes. Les méthodes de synthèse totale du squelette naphthyridine-2,7 au départ de produits ouverts sont encore plus complexes. Ainsi B.M. ISELIN et K. HOFFMANN J. Am. Chem. Soc. 76 (1954) pages 3220 à 3222 ont préparé des dérivés à structure naphthyridinique à partir du glutarate de diéthyle et de l'éthyl- ou du phénylmalonitrile par un procédé qui implique dans une première phase la formation d'un ß-(éthyl- ou phényldicyanométhyl)glutarate de diéthyle puis la cyclisation de ce dernier par l'acide sulfurique avec formation d'un composé tétra-oxoperhydronaphthyridinique, qui est réduit ensuite en dérivé de perhydronaphthyridine-2,7 par l'aluminohydrure de lithium. Enfin C. JUTZ et al Ber. 99 2484 (1966) ont obtenu la formyl-4 naphthyridine-2,7 par cyclisation du triperchlorate de diméthylamino-1 diméthylimonio-5 diméthylimonio- méthyl-2 [ß-diméthylamino, ß'-diméthylimonioisopropènyl]-3 pentadiène-1,3 issu lui-même de la condensation du diméthylformamide avec l'isobutène en présence d'oxychlorure de phosphore ($POCl_3$).

En définitive l'industrie ne dispose pas de moyens simples pour accéder à la structure naphthyridinique-2,7 à partir de matières premières courantes.

La présente invention a précisément pour objet de mettre à la disposition de l'industrie un procédé de préparation de naphthyridines-2,7 à partir de composés organiques usuels.

Un autre objet de la présente invention réside dans de nouvelles polyalkylnaphthyridines-2,7.

Plus spécifiquement, la présente invention a pour objet des naphthyridines-2,7 caractérisées en ce qu'elles répondent à la formule générale :

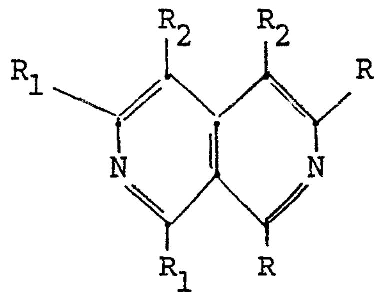

(I)

dans laquelle :

- les symboles $R_1$, qui sont identiques, représentent des radicaux alkyles linéaires ou ramifiés, ne comportant pas dans ce dernier cas d'atome de carbone tertiaire juxtanucléaire, ayant de 1 à 20 atomes de carbone, éventuellement substitués par un ou plusieurs radicaux aromatiques ou hétérocycliques ou par un ou plusieurs groupes fonctionnels situés au moins en position $\gamma$ par rapport au carbone cyclique du cycle naphthyridine
- l'un quelconque au moins des symboles R représente un radical $R_1$ tel que défini ci-avant et, le cas échéant, l'autre symbole R représente un radical $R_3$ défini comme un radical alkyle linéaire ou ramifié ayant de 1 à 20 atomes de carbone, éventuellement substitué par un ou plusieurs radicaux aromatiques ou hétérocycliques ou par un ou plusieurs groupes fonctionnels situés au moins en position $\gamma$ par rapport au carbone cyclique du cycle naphthyridine, et qui peut être identique ou différent des radicaux $R_1$.
- les symboles $R_2$ représentent un atome d'hydrogène ou un radical méthyle avec la restriction que l'un quelconque au moins des symboles $R_2$ représente un atome d'hydrogène.

La présente invention a encore pour objet un procédé de préparation des polyalkylnaphthyridines-2,7 de formule (I), caractérisé en ce qu'il comprend :

A - une première étape au cours de laquelle on fait réagir en milieu anhydre, en présence d'un acide de Lewis comme catalyseur, un agent acylant de formule générale :

$$R_1 - \overset{O}{\overset{\|}{C}} - Z \qquad (II)$$

dans laquelle :

. $R_1$ a la définition donnée ci-avant,

. Z représente un atome d'halogène

avec un composé éthylénique de formule générale :

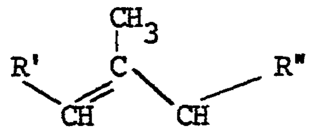

(III)

dans laquelle l'un quelconque des radicaux R' et R" représente un atome d'hydrogène ou le radical méthyle et l'autre un atome d'hydrogène ou un groupe alkylcarbonyle $R_3$ - CO - où $R_3$ a la signification donnée ci-avant, ou avec un composé susceptible de conduire à la formation "in situ" d'un composé éthylénique de formule (III) dans les conditions de la réaction et répondant à la formule générale :

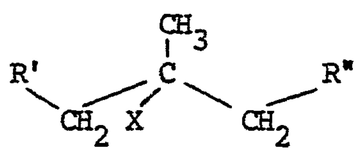

(IV)

dans laquelle :

. R' et R" ont la signification donnée ci-avant ;

. X représente : un atome d'hydrogène ; un atome d'halogène ; un reste de formule $R_6O$- où $R_6$ est un atome d'hydrogène ou un radical alkyle ; un reste ester Q - O - où Q représente le reste d'un acide minéral ou carboxylique ou sulfonique ; un radical isobutyle ou isobutènyle avec la restriction que R' et R" représentent dans ce cas un atome d'hydrogène.

B - une seconde étape au cours de laquelle la masse réactionnelle provenant de la première étape est traitée, en absence d'eau, par de l'ammoniac anhydre ou un sel d'ammonium, éventuellement après élimination de l'agent acylant et/ou du composé éthylénique ou de son précurseur en excès.

On a constaté de manière surprenante qu'en traitant les produits d'acylation des isooléfines ou de leurs précurseurs par de l'ammoniac ou des sels d'ammonium en absence d'eau la réaction conduit le plus souvent à la formation prépondérante des naphthyridines de formule (I) alors qu'il est bien connu que lorsqu'on traite les produits d'acylation des isooléfines ou de leurs précurseurs par de l'ammoniaque on aboutit à des pyridines substituées. Il se forme également à côté des polyalkylnaphthyridines des trialkylpyridines.

Comme exemples de groupes aromatiques ou hétérocycliques éventuellement portés par les radicaux alkyles $R_1$ ou $R_3$ définis précédemment on peut citer des radicaux phényle, toluyle, naphtyle, pipéridinyle, pyridinyle. Lorsque $R_1$ et/ou $R_3$ sont substitués par un ou plusieurs groupes fonctionnels, ces derniers sont choisis parmi ceux qui sont inertes dans les conditions réactionnelles propres à chaque étape du procédé. Comme exemples de groupes fonctionnels répondant à cette exigence on peut citer les groupes alkyl-, cycloalkyl-, arylcarbonyloxy, les groupes alkyloxycarbonyle, nitro, halogéno primaire (chloro, bromo, fluoro), oxo. A titre d'exemples non limitatifs de radicaux $R_1$ et $R_3$ on peut se référer aux radicaux méthyle, éthyle, n-propyle, isopropyle, n-butyle, n-pentyle, isopentyle, n-hexyle, méthyl-4 pentyle, n-heptyle, n-décyle, dodécyle, octadécyle, chloro-4 butyle, phényl-4 butyle, (pyridinyl-2)-4 butyle.

De préférence : les radicaux $R_1$ et $R_3$ représentent des radicaux alkyles ayant de 1 à 10 atomes de carbone et plus préférentiellement encore des radicaux alkyle ayant de 1 à 5 atomes de carbone ; dans la formule (II) Z représente un atome de chlore ou de brome et dans la formule (IV), X représente un atome de chlore, de brome, un radical hydroxyle, un radical méthoxy, un radical méthylcarbonyloxy, un radical p-tolylsulfonyloxy. R' et R" représentent de préférence un atome d'hydrogène ou un radical acyle tel que défini ci-avant.

Comme exemple de composés de formules générales (III) et (IV) pouvant être utilisés dans le procédé selon la présente invention on peut citer l'isobutène, le méthyl-2 butène-1, le méthyl-2 butène-2, le triméthyl-2,4,4 pentène-1, le triméthyl-2,4,4 pentène 2 ou leurs mélanges, l'oxyde de mésityle, la méthyl-4 pentène-4 one-2, la

diméthyl-3,4 pentène-3 one-2, la diméthyl-3,4 pentène-4 one-2, la méthyl-4 hexène-3 one-2, la méthyl-5 hexène-4 one-3, la diméthyl-2,5 hexène-4 one-3, le t-butanol, le chlorure de t-butyle, le bromure de t-butyle, le chloro-2 méthyl-2 butane, le méthyl-2 butanol-2, la méthyl-4 hydroxy-4 pentanone-2 (diacétone-alcool), la méthyl-4 méthoxy-4 pentanone-2 (pentoxone), le méthoxy-2 méthyl-2 propane, l'acétate de t-butyle, le tosylate de t-butyle, l'isobutane, l'isopentane, le triméthyl-2,2,4 pentane (isooctane).

Lorsqu'on fait appel à des isoparaffines telles que l'isobutane, l'isopentane ou l'isooctane on peut, sans sortir du cadre de la présente invention, mettre en oeuvre conjointement à ces dernières un composé donnant naissance à un carbocation, plus facilement que l'isoparaffine dans les conditions de la première étape, lequel carbocation permet à son tour par un transfert d'hydrure à partir de l'isoparaffine la formation d'un carbocation engendrant "in situ" une isooléfine [cf. L. SCHMERLING J. Am. Chem. Soc. 97 (1975 6134-6136 ; C. NDANDJI Thèse Marseille (1977) ; demande de brevet français No. 76-27 225 publiée sous le No. 2 364 190 ; G. OLAH, Friedel Crafts and Related Reactions Volume II (1963) page 1111 ; M. ARNAUD Thèse Marseille (1980) pages 114 à 118]. Comme exemple de composés activant le passage des isoparaffines aux isooléfines on peut citer des halogénures d'alkyles ou des alcools tels que ceux cités dans la demande de brevet No. 76-27 225. Plus particulièrement on fait appel à des halogénures secondaires ou tertiaires ou à des alcools secondaires ou tertiaires.

Comme composés de formule (III) ou (IV) on fait appel de préférence à l'isobutène,au diisobutylène, au chlorure de t-butyle, au t-butanol, au diacétone-alcool, à l'oxyde de mésityle, à la pentoxone, à la méthyl-5 hexène-4 one-3 et à la diméthyl-2,5 hexène-4 one-3.

Parmi les agents acylants de formule (I), on peut utiliser des halogénures d'alcanoyles tels que le chlorure d'acétyle, le bromure d'acétyle, le chlorure de propionyle, le chlorure d'isobutyryle, le chlorure de valéryle, le chlorure de butyryle.

Les acides de Lewis auxquels on fait appel pour réaliser la première étape du procédé selon la présente invention sont ceux utilisés dans les réactions de Friedel et Crafts, et notamment dans les réactions

d'acylation [cf. G. OLAH, Friedel-Crafts and Related Reactions Volume 1 (1963) pages 201 à 291]. Il s'agit essentiellement d'halogénures de métaux des groupes 3a, 4a et 5a et des métaux de transition et notamment des groupes 2b, 4b, 7b, 8 de la classification périodique des éléments [R.C. WEAST, Handbook of Chemistry and Physics 53ème édition (1972-1973)]. Plus spécifiquement on peut citer les halogénures de Ti, Fe, Zn, Al, Sn et Sb et notamment le tétrachlorure de titane, le trichlorure de titane, le tétrabromure de titane, le tribromure de titane, le chlorure ferrique, le bromure ferrique, le chlorure de zinc, le chlorure d'aluminium, le bromure d'aluminium, le fluorure d'aluminium, le tétrachlorure d'étain, le pentachlorure d'antimoine. Parmi ces halogénures métalliques on utilise de préférence les chlorure et bromure d'aluminium.

La première étape du procédé selon l'invention peut être conduite en l'absence de tout solvant ou en présence d'un diluant organique inerte dans les conditions de la réaction. les solvants auxquels on peut avoir recours sont ceux généralement utilisés dans les réactions d'acylation en présence d'acides de Lewis. On peut citer notamment le sulfure de carbone, le nitrobenzène, les nitroalkanes (nitrométhane, nitroéthane, nitropropanes) et des hydrocarbures halogénés aliphatiques ou aromatiques (dichloroéthane, dichlorométhane, chloroforme, dichlorobenzène). En pratique, il est préférable d'opérer dans un excès d'agent acylant.

Sans que la portée de l'invention soit limitée en quoi que ce soit par la référence à un mécanisme réactionnel particulier, il apparaît probable que la première étape du procédé consiste en une tétraacylation ou en une triacylation du composé de départ de formule (III) préformé ou formé "in situ" à partir du précurseur de formule (IV) selon que ceux-ci ne comportent pas ou bien comporte déjà un radical acyle de formule $R_3$ - CO, avec formation d'un composé tétracétonique intermédiaire. Lorsque les composés éthyléniques ou leurs précurseurs répondent aux formules (III) et (IV) dans lesquelles R' et R" représentent de l'hydrogène, ces composés tétracétoniques conduisent à la formation de tétraalkyl-1,3,6,8 naphthyridine-2,7 de formule générale (I) dans laquelle les radicaux R représentent tous les deux un radical $R_1$.

Lorsque dans les formules (III) et (IV) l'un des radicaux R' et R" représente un radical méthyle et l'autre un atome d'hydrogène, le procédé selon l'invention conduit à des tétraalkyl-1,3,6,8 méthyl-4 naphthyridines. Lorsqu'on fait appel à des composés éthyléniques ou à leurs précurseurs de formules (III) et (IV) dans lesquelles l'un des radicaux R' et R" représente un atome d'hydrogène et l'autre un reste acyle de formule $R_3 - CO$, le procédé selon l'invention conduit, quand $R_3$ est différent du radical $R_1$ de l'agent acylant, à trois types de naphthyridines. Ainsi, lorsqu'on met en oeuvre d'une part un agent acylant de formule $R_1 - \overset{O}{\overset{||}{C}} - Z$ et des composés de formules :

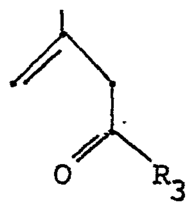
(III)'

ou

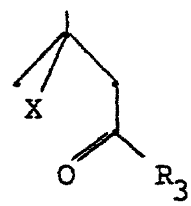
(IV)'

dans lesquelles $R_1$ est différent de $R_3$, le procédé selon l'invention conduit :

a) à des tétraalkylnaphthydridines-2,7 non symétriques isomères de formules générales :

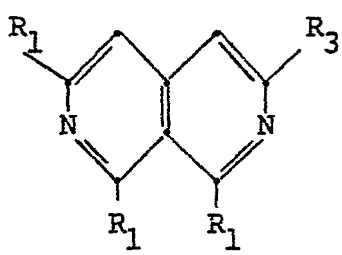
(V)

et

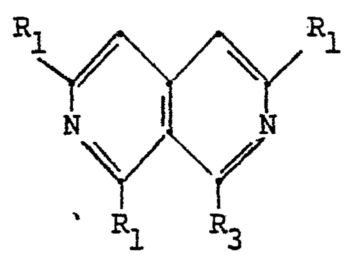
(VI)

résultant de la triacylation des composés (III)' ou (IV)' et formées de façon prépondérante ;

b) à la formation minoritaire des tétraalkyl-1,3,6,8 naphthyridines-2,7 symétriques issus de la tétraacylation de composés éthyléniques intermédiaires formés "in situ" par désacylation des

produits de départ (III)' ou (IV)' dans le milieu réactionnel.

Naturellement si l'un des radicaux R' et R" est un radical méthyle et l'autre un radical $R_3 - \overset{O}{\overset{\|}{C}}$ le procédé aboutit à des tétraalkylméthylnaphthyridine-2,7 disymétriques lorsque $R_1$ et $R_3$ sont différents.

Parmi les nouvelles naphthyridines selon la présente invention, on peut citer :

- la tétraméthyl-1,3,6,8 naphthyridine-2,7,
- la tétraéthyl-1,3,6,8 naphthyridine-2,7,
- la tétraisopropyl-1,3,6,8 naphthyrine-2,7,
- la méthyl-3 triéthyl-1,6,8 naphthyridine-2,7,
- la méthyl-1 triéthyl-3,6,8 naphthyridine-2,7,
- la pentylméthyl-1,3,4,6,8 naphthyrydine-2,7,
- la tétra-n-butyl-1,3,6,8 naphthyridine-2,7,
- la tétra-n-propyl-1,3,6,8 naphthyridine-2,7,
- l'éthyl-3 triméthyl-1,6,8 naphthyridine-2,7,
- l'éthyl-1 triméthyl-3,6,8 naphthyridine-2,7,
- l'isopropyl-3 triméthyl- 1,6,8 naphthyridine-2,7.

La quantité d'agent acylant exprimée en moles par mole de composé éthylénique de formule (III) ou de précurseur de composé éthylénique de formule (IV) peut varier dans de larges limites en fonction de la nature de ces composés. En pratique il est préférable que cette quantité d'agent acylant soit au moins égale à celle théoriquement nécessaire à la polyacylation du composé éthylénique de formule (III) ou de son précurseur, c'est-à-dire à 3 ou à 4 moles par mole selon que l'un des radicaux R' et R" représente ou non un groupe $R_3 - \overset{O}{\overset{\|}{C}} -$. En définitive, il est préférable que la quantité d'agent acylant soit d'au moins environ trois moles par mole de composé éthylénique ou de son précurseur et plus préférentiellement encore d'au moins environ 4 moles par mole de composé éthylénique ou de précurseur. Le recours à des quantités d'agent acylant inférieures à 3 moles par mole de composé éthylénique ou de précurseur se traduit rapidement par une baisse du taux de transformation et des rendements en naphthyridines. Il n'y a pas de

limite supérieure critique du rapport molaire agent acylant/composé éthylénique ou précurseur, l'agent acylant pouvant être utilisé comme milieu réactionnel. On a en outre constaté qu'un excès d'agent acylant est bénéfique pour le déroulement de la réaction. En pratique ce rapport molaire est de préférence compris entre 6 et 20. L'excès d'agent d'acylation n'est pas gênant puisque ce composé peut être récupéré avant le traitement à l'ammoniac par tout moyen approprié par exemple par distillation.

La quantité d'acide de Lewis exprimée en mole par mole de composé éthylénique de formule (III) ou de précurseur de formule (IV) peut varier dans de larges limites. En règle générale elle est d'au moins 0,5 mole et de préférence au moins 2 moles d'acide de Lewis par mole de composé éthylénique ou de son précurseur. De préférence ce rapport molaire est compris entre environ 2 et environ 6. On peut évidemment sortir des limites définies précédemment mais sans que cela se traduise par des avantages particuliers. Ainsi ce rapport peut être abaissé jusqu'à une valeur d'environ 0,1 mais alors le taux de transformation de l'isooléfine ou de son précurseur est fortement abaissé.

La température à laquelle la première étape du procédé selon l'invention est réalisée peut être comprise entre environ -10 et environ 100°C et de préférence entre environ 20 et environ 50°C. On pourrait cependant opérer en dehors de ces limites sans sortir pour autant du cadre de la présente invention. Bien qu'il ne soit généralement pas nécessaire de conduire la première étape du procédé selon l'invention sous pression, il peut être avantageux lorsqu'un des réactifs est gazeux dans les conditions de la réaction d'opérer sous pression autogène. Lorsqu'on fait appel à l'isobutène comme composé de départ on peut le faire barboter au sein d'une solution de l'acide de Lewis dans l'agent acylant maintenue sous agitation.

A l'issue de la première étape d'acylation dont la durée ne présente aucun caractère critique, le produit d'acylation intermédiaire est soumis à un traitement par l'ammoniac anhydre ou par un sel d'ammonium anhydre. La masse réactionnelle résultant de la première phase peut être soumise directement à ce traitement ou bien on peut la débarrasser au préalable, en totalité ou en partie, des produits de

départ non transformés et en particulier de l'agent acylant en excès. A cet effet on peut faire appel aux moyens usuels de séparation ; on peut par exemple procéder à une distillation sous vide.

Pour traiter le mélange réactionnel résultant de la phase d'acylation on peut faire appel soit à de l'ammoniac liquide soit à de l'ammoniac gazeux. La quantité d'ammoniac exprimée en moles par mole de composé éthylénique ou de son précurseur peut varier dans de larges limites. De préférence ce rapport molaire est d'au moins deux mais en pratique on opère en présence d'une quantité d'ammoniac suffisante pour transformer en amide l'agent acylant mis en oeuvre si celui-ci n'a pas été éliminé auparavant. En définitive il n'y a pas de valeur supérieure critique du rapport molaire $NH_3$/composé éthylénique ou précurseur. On pourrait donc faire appel à des valeurs de ce rapport supérieures à 50 sans sortir du cadre de la présente invention. Lorsqu'on fait appel à l'ammoniac liquide on ajoute progressivement la masse réactionnelle d'acylation à l'ammoniac maintenu à la température adéquate. Au lieu d'utiliser l'ammoniac liquide on peut faire arriver un courant d'ammoniac gazeux au sein de la masse réactionnelle d'acylation éventuellement diluée par un solvant inerte bien que ce mode opératoire ne soit pas aussi satisfaisant que celui mettant en oeuvre l'ammoniac liquide. Dans ce cas on peut faire appel à des composés organiques liquides dans les conditions du traitement tels que les alcools, les éthers, les hydrocarbures aliphatiques halogénés, les nitroalcanes, les nitriles. On peut citer par exemple le méthanol, l'éthanol, l'oxyde d'éthyle, le chloroforme, le chlorure de méthylène, le dichloro-1,2 éthane, le nitrométhane, l'acétonitrile, le propionitrile.

Selon un autre mode de réalisation de l'invention l'ammoniac peut être remplacé par un sel d'ammonium. En pareil cas on fait appel à des sels représentés par le formule générale :

$$A^{n-}(NH_4^+)_n \qquad (VII)$$

dans laquelle $A^{n-}$ représente le reste d'un acide minéral ou organique et n un nombre entier supérieur ou égal à 1 et, de préférence égal à 1 ou 2.

Plus spécifiquement, on fait appel comme sels d'ammonium à des sels d'acides mono- ou polycarboxyliques tels que les acides formique, acétique, propionique, butyrique, benzoïque, toluique, succinique, hexanoïque, adipique cités à titre non limitatif. On utilise de préférence les formiate, acétate et propionate d'ammonium qui sont les plus courants. Parmi les sels d'ammonium d'acides minéraux on peut citer les chlorure, bromure, carbonate et phosphates d'ammonium. On pourrait, sans sortir du cadre de la présente invention, faire appel à des sels d'ammonium des acides sulfoniques aliphatiques, cycloaliphatiques ou aromatiques tels que l'acide méthanesulfonique, l'acide benzènesulfonique ou l'acide p-toluènesulfonique.

Lorsqu'on fait appel à un sel d'ammonium on peut recourir aux mêmes diluants organiques que ceux cités pour le traitement par l'ammoniac ; les alcanols, les nitroalcanes et les nitriles aliphatiques conviennent tout particulièrement bien.

La quantité de sel d'ammonium exprimée en mole par mole de composé éthylénique de formule (III) ou de précurseur de formule (IV) peut varier dans de larges limites. De préférence le rapport molaire sel d'ammonium/composé éthylénique ou précurseur est au moins égal à environ 2. Il n'y a pas de valeur limite supérieure critique de ce rapport mais en pratique il n'est pas utile qu'il soit supérieur à 10. De préférence ce rapport est compris entre 2,5 et 8. Lorsque le sel d'ammonium est utilisé par commodité au sein d'un diluant organique la concentration du sel dans ce diluant n'est pas critique. Toutefois pour limiter le volume de la masse réactionnelle et atteindre une bonne productivité de l'appareillage, il est préférable de faire appel à des solutions concentrées de sel dans le liquide organique choisi. Ainsi on peut mettre en oeuvre des solutions saturées en sel, voire même des suspensions de sel d'ammonium aisément manipulables.

La température à laquelle est effectué le traitement de la masse réactionnelle résultant de la première étape d'acylation dépend de la nature de l'agent de traitement et des conditions du traitement. Ainsi lorsqu'on fait appel à l'ammoniac liquide cette température peut se situer au voisinage de la température d'ébullition de l'ammoniac à pression normale, c'est-à-dire aux environs de -33°C. On peut toutefois

opérer également à des températures notablement inférieures, de l'ordre de -60°C. Lorsqu'on utilise un flux d'ammoniac gazeux ou un sel d'ammonium, il n'est pas nécessaire d'avoir recours à des températures aussi basses ; en général des températures comprises entre -20 et + 20°C conviennent bien. En définitive, en fonction des conditions du traitement final, la température peut être comprise entre -60°C et + 20°C et de préférence entre -30 et + 20°C. Naturellement ce traitement peut être réalisé à pression normale ou sous pression plus élevée, notamment lorsqu'on fait appel à l'ammoniac, ce qui en pareil cas permet d'opérer avec de l'ammoniac liquide à des températures supérieures à -30°C.

Lorsque le traitement final par l'ammoniac ou les sels d'ammonium est terminé, la masse réactionnelle résultant de cette seconde étape est traitée pour séparer les produits de la réaction et notamment les naphthyridines. Selon un mode opératoire préféré la masse réactionnelle est traitée à l'eau, éventuellement après élimination de l'ammoniac en excès, puis la totalité de la masse hydroorganique obtenue est extraite au moyen d'un solvant organique non miscible à l'eau si un tel solvant n'a pas été mis en oeuvre au cours de l'une et/ou l'autre des deux étapes du procédé. Cette extraction permet de séparer les produits organiques et notamment les composés hétérocycliques et les alcanamides dérivés de l'agent acylant en excès des produits minéraux et éventuellement du sel d'ammonium en excès. La solution organique ainsi obtenue est traitée à son tour pour séparer les composés hétérocycliques azotés des alcanamides. Un moyen commode pour réaliser cette séparation consiste à chasser le solvant d'extraction par évaporation à sec puis à traiter le résidu ainsi obtenu par une solution aqueuse alcaline pour saponifier les alcanamides, puis à extraire la masse réactionnelle de la saponification par un solvant approprié pour séparer les composés hétérocycliques. Les polyalkylnaphthyridines peuvent être à leur tour séparées des alkylpyridines se formant dans la réaction par tout moyen adéquat, par exemple par distillation ou chromatographie en phase liquide. La nature des solvants convenant à ces divers traitements peut être déterminée au moyens d'essais simples dans chaque cas particulier. Les chloroalcanes tels que le chloroforme constituent des exemples de solvants d'extraction qui conviennent tout particulièrement bien.

Les nouvelles polyalkylnaphthyridines-2,7 qui constituent un des objets de la présente invention peuvent être aisément transformées en dérivés fonctionnalisés par les méthodes classiques utilisées en chimie organique. Ainsi par exemple un ou plusieurs des groupes alkyles et notamment méthyle peuvent être oxydés en groupes aldéhyde ou acide carboxylique qui peuvent à leur tour être réduits en les alcools correspondants. Les composés ainsi obtenus peuvent constituer des intermédiaires précieux pour la synthèse de produits actifs à structure naphthyridinique-2,7.

Les exemples suivants illustrent l'invention et montrent comment elle peut être mise en pratique.

EXEMPLE 1

Dans un réacteur cylindrique en verre de 400 ml à double enveloppe pour circulation d'un liquide de refroidissement ou de chauffage, équipé d'un réfrigérant ascendant dans lequel on fait circuler de l'alcool refroidi à -30°C, d'un agitateur à vibration et d'une ampoule de coulée, on charge 0,3 mole de chlorure d'aluminium. On refroidit le contenu du réacteur à 0°C par circulation d'eau glacée dans la double enveloppe puis on ajoute successivement, sous agitation, en 5 minutes 1,6 mole de chlorure d'acétyle puis 0,1 mole de t-butanol en 30 minutes. On observe un abondant dégagement d'acide chlorhydrique. On porte, dès la fin de l'addition du t-butanol, le contenu du réacteur à 35°C et on maintient 30 minutes dans ces conditions.

Le mélange réactionnel résultant de l'acylation est ensuite refroidi à 0°C puis ajouté sous agitation en 20 minutes à 500 ml d'ammoniac liquide refroidi par un bain à -60°C. En raison de l'exothermicité de la réaction, la température de la masse réactionnelle s'élève progressivement et atteint -30°C à la fin de l'addition. La masse réactionnelle obtenue est alors amenée à la température de 20°C, ce qui provoque l'élimination de l'ammoniac par évaporation, puis on lui ajoute 250 ml d'eau à 20°C sous agitation. La solution aqueuse issue de ce traitement est extraite en continu par 150 ml de chloroforme dans un extracteur liquide-liquide. On obtient de cette façon 150 ml d'une solution organique et 250 ml d'une phase aqueuse qui contient de

l'hydroxyde d'aluminium et des aluminates d'ammonium.

La phase chloroformique est évaporée à sec et le résidu organique est repris par 100 ml d'une solution aqueuse d'acide chlorhydrique à 10 % en poids. La phase aqueuse ainsi obtenue est lavée 2 fois par 40 ml de chloroforme, puis amenée à pH supérieur à 7 par addition de 20 ml d'une solution aqueuse de soude à 36°bé. La phase aqueuse alcaline est alors extraite au chloroforme dans un extracteur liquide-liquide. La phase chloroformique ainsi obtenue est évaporée à sec et le résidu obtenu est repris par 50 ml d'oxyde d'éthyle à 20°C. Une partie insoluble résiduelle est séparée de l'extrait éthéré par filtration, lavée par 2 fois 10 ml d'éther, séchée et identifiée comme étant de l'acétamide.

La solution éthérée à laquelle a été ajouté l'éther de lavage est évaporée à sec. On recueille ainsi 14,7 g d'un résidu dans lequel on dose par résonance magnétique nucléaire 63 % en mole de tétraméthyl-1,3,6,8 naphthyridine-2,7 et 37 % en mole de triméthyl-2,4,6 pyridine, ce qui représente respectivement 57 % et 34 % du t-butanol engagé à l'étape d'acylation.

La naphthyridine a été séparée de la pyridine de la manière suivante :

On ajoute 50 ml d'une solution aqueuse d'acide chlorhydrique à 10 % en poids au résidu obtenu précédemment puis on provoque le relargage de la tétraméthylnaphthyridine à partir de pH = 3,5 par addition à la solution aqueuse chlorhydrique d'une solution aqueuse de soude. La naphthyridine est isolée par filtration. La triméthylpyridine est séparée par extraction du filtrat aqueux par du chloroforme, évaporation à sec du solvant et distillation sous pression réduite à 20 mm de mercure.

Les deux composés obtenus à l'issue de ce traitement présentent des spectres de résonnance magnétique nucléaire du proton (RMN) et de masse (SM) qui ont les caractéristiques suivantes :

1°) tétraméthyl-1,3,6,8 naphthyridine-2,7 :

- RMN à 60 MHz dans le chloroforme deutéré :
  . singulet (6 H) à 2,6 ppm (partie par million)

. singulet (6 H) à 3,05 ppm

. singulet (2 H) à 7,1 ppm

- SM à 80 eV : m/e 187 (16,2) ; 186 (100 ; 185 (48,4) ; 171 (12,2) ; 144 (6,7) ; 115 (8,5) ; 77 (8,4) ; 51 (7,9) ; 42 (8,1) ; 39 (11,2).

2°) triméthyl-2,4,6 pyridine :

- RMN à 60 MHz dans le chloroforme deutéré :

. singulet (6 H) à 2,2 ppm

. singulet (6 H) à 2,45 ppm

. singulet (2 H) à 6,78 ppm

- SM à 80 eV : m/e 122 (13,8) ; 121 (100) ; 120 (42,3) ; 106 (16,5) ; 79 (18,9) ; 77 (10,1) ; 39 (16).

EXEMPLE 2

On opère dans les conditions décrites à l'exemple 1 en remplaçant à l'étape d'acylation le t-butanol par le chlorure de t-butyle. Le traitement du mélange d'acylation par l'ammoniac puis de la masse réactionnelle de la deuxième étape sont réalisés comme décrit à l'exemple 1. On a recueilli de cette façon 15,81 g d'un mélange contenant 65 % en mole de tétraméthyl-1,3,6,8 naphthyridine-2,7 et 35 % en mole de triméthyl-2,4,6 pyridine, ce qui représente respectivement 63 % et 33 % du chlorure de t-butyle engagé à l'étape d'acylation.

EXEMPLE 3

La première étape d'acylation du t-butanol est conduite comme à l'exemple 1 en remplaçant le chlorure d'acétyle par le chlorure de propionyle. La masse réactionnelle d'acylation est traitée par l'ammoniac liquide de la façon décrite à l'exemple 1. Le mélange réactionnel est hydrolysé et l'hydrolysat est extrait au chloroforme comme à l'exemple 1.

La phase chloroformique est évaporée à sec et le résidu organique est repris par 200 ml d'une solution aqueuse de soude à 10 % en poids. Cette phase aqueuse est chauffée 3 heures à 60°C pour saponifier la n-propionamide, puis refroidie à 20°C et extraite par 3 fois 30 ml de

chloroforme.

Le nouvel extrait chloroformique est évaporé à sec et on recueille 18,9 g d'un résidu dans laquel on a dosé comme à l'exemple 1 :

- 74 % en mole de tétraéthyl-1,3,6,8 naphthyridine-2,7,
- 26 % en mole de diéthyl-2,6 méthyl-4 pyridine,

ce qui représente respectivement 64 % et 23 % du t-butanol engagé à l'étape d'acylation.

Les composés obtenus possèdent des spectres de RMN et de masse ayant les caractéristiques suivantes :

a) tétraéthyl-1,3,6,8 naphthyridine-2,7 :

- RMN à 250 MHz dans $CDCl_3$
  . triplet (6 H) à 1,36 ppm
  . triplet (6 H) à 1,4 ppm
  . quartet (4 H) à 2,9 ppm
  . quartet (4 H) à 3,41 ppm
  . singulet (2 H) à 7,16 ppm
- SM à 80 eV : m/e 243 (7,3) ; 242 (43,4) ; 241 (18,9) ; 228 (12,1) ; 227 (80,2) ; 214 (19,1) ; 213 (100) ; 212 (17,6) ; 211 (13,2) ; 197 (7,4) 106 (10,6).

b) diéthyl-2,6 méthyl-4 pyridine :

- RMN à 250 MHz dans $CDCl_3$
  . singulet (6 H) à 1,25 ppm
  . singulet (3 H) à 2,25 ppm
  . quartet (4 H) à 2,74 ppm
  . singulet (2 H) à 6,8 ppm
- SM à 80 eV : m/e 150 (4,8) ; 149 (47,3) ; 148 (100) ; 133 (14,7) ; 121 (27,3) ; 106 (7,2) ; 91 (4) ; 77 (5,3) ; 39 (10,3).

<u>EXEMPLE 4</u>

On opère comme à l'exemple 1 en remplaçant à la phase d'acylation le chlorure d'acétyle par le chlorure d'isobutyryle puis en

traitant le mélange d'acylation par l'ammoniac liquide comme à l'exemple 1. La masse réactionnelle résultant de cette phase est traitée selon le mode opératoire de l'exemple 3. On a recueilli 18,3 g d'un mélange dans lequel on a dosé :

- 60 % en mole de tétraisopropyl-1,3,6,8 naphthyridine-2,7,
- 40 % en mole de diisopropyl-2,6 méthyl-4 pyridine,

ce qui représente respectivement 44 % et 29 % du t-butanol engagé à l'étape d'acylation.

Les composés obtenus possèdent des spectres RMN et de masse ayant les caractéristiques suivantes :

1°) tétraisopropyl-1,3,6,8 naphthyridine-2,7 :

- RMN à 250 MHz dans $CDCl_3$
  - . doublet (12 H) à 1,34 ppm
  - . doublet (12 H) à 1,40 ppm
  - . septuplet ( 2 H) à 3,06 ppm
  - . septuplet ( 2 H) à 3,92 ppm
  - . singulet ( 2 H) à 7,1 ppm
- SM à 80 eV : m/e 299 (7,9) ; 298 (27) ; 297 (6) ; 284 (12,5) ; 283 (46,1) ; 270 (9,4) ; 269 (19,5) ; 256 (23,8) ; 255 (100) ; 225 (6,7).

2°) diisopropyl-2,6 méthyl-4 pyridine :

- RMN à 250 MHz dans $CDCl_3$
  - . doublet (12 H) à 1,28 ppm
  - . singulet ( 3 H) à 2,28 ppm
  - . septuplet ( 2 H) à 3 ppm
  - . singulet ( 2 H) à 6,8 ppm
- SM à 80 eV : m/e 177 (3,7) ; 176 (14,6) ; 162 (39) ; 150 (11,4) ; 149 (100) ; 134 (8,7) ; 91 (6,2) ; 77 (6,5) ; 41 (10,4).

EXEMPLE 5

Dans l'appareil décrit à l'exemple 1, on charge 0,3 mole de

chlorure d'aluminium que l'on refroidit à 0°C puis on ajoute, sous agitation, 1 mole de chlorure de propionyle en 2 minutes puis 0,1 mole de méthyl-4 méthoxy-4 pentanone-2 en 30 minutes. On constate un dégagement d'acide chlorhydrique. Lorsque l'addition est achevée on laisse remonter la température à 35°C et maintient 1 heure dans ces conditions.

Le mélange réactionnel résultant de l'acylation est traité par 400 ml d'ammoniac liquide de la manière décrite à l'exemple 1. La masse résultant de ce traitement fait l'objet des opérations décrites à l'exemple 3.

On a recueilli au cours de ce traitement 14,6 g d'un mélange dans lequel on a dosé par RMN :

a) 53,9 % en mole de méthyl-3 triéthyl-1,6,8 naphthyridine-2,7
b) 17,7 % en mole de tétraéthyl-1,3,6,8 naphthyridine-2,7
c) 5,4 % en mole de méthyl-1 triéthyl-3,6,8 naphthyridine-2,7
d) 19,1 % en mole de diméthyl-2,4 éthyl-6 pyridine
e) 3,9 % en mole de diéthyl-2,6 méthyl-4 pyridine,

ce qui correspond respectivement à 38 %, 12 %, 4 %, 13 % et 3 % de la pentoxone engagée à l'étape d'acylation.

Les composés identifiés présentent des spectres RMN et de masse ayant les caractéristiques suivantes :

a) Méthyl-3 triéthyl-1,6,8 naphthyridine-2,7 :

- RMN à 250 MHz dans $CDCl_3$
  . triplet (3 H) à 1,36 ppm
  . triplet (6 H) à 1,40 ppm
  . singulet (3 H) à 2,61ppm
  . quartet (2 H) à 2,90 ppm
  . quartet (4 H) à 3,41 ppm
  . singulet (1 H) à 7,14 ppm
  . singulet (1 H) à 7,16 ppm
- SM à 80 eV : m/e 229 (11) ; 228 (60,7) ; 227 (17,6) ; 214 (13,9) ; 213 (93,6) ; 211 (10,9) ; 200 (14,6) ; 199 (100) ; 198 (20,4) ; 197 (13,5) ; 184 (9,3) ; 183 (7,3) ; 106 (10,7) ; 99 (9,9).

b) tétraéthyl-1,3,6,8 naphthyridine-2,7 :

Le composé isolé possède un spectre RMN et de masse dont les caractéristiques sont celles indiquées à l'exemple 3.

c) méthyl-1 triéthyl-3,6,8 naphthyridine-2,7 :

- RMN à 250 MHz dans $CDCl_3$
  . triplet (6 H) à 1,3 ppm
  . triplet (3 H) à 1,40 ppm
  . singulet (3 H) à 3,1 ppm
  . quartet (4 H) à 2,90 ppm
  . quartet (2 H) à 3,41 ppm
  . singulet (2 H) à 7,16 ppm
- SM à 80 eV : m/e 229 (6,3) ; 228 (29,5) ; 227 (37,9) ; 214 (12,3) ; 213 (100) ; 212 (6,1) ; 211 (8,4) ; 199 (11) ; 113 (5,9) ; 106 (5,3).

d) diméthyl-2,4 éthyl-6 pyridine :

- RMN à 250 MHz dans $CDCl_3$
  . triplet (3 H) à 1,25 ppm
  . singulet (3 H) à 2,25 ppm
  . singulet (3 H) à 2,45 ppm
  . quartet (2 H) à 2,74 ppm
  . singulet (2 H) à 6,8 ppm
- SM à 80 eV : m/e 136 (6,7) ; 135 (70,5) ; 134 (100) ; 108 (6,4) ; 107 (38,8) ; 106 (16,6) ; 77 (8,8) ; 53 (7,8) ; 41 (7,2 ; 39 (16,7) ;

e) diéthyl-2,6 méthyl-4 pyridine :

Le composé isolé présente des spectres RMN et de masse identiques à celui du composé b) de l'exemple 3.

EXEMPLE 6

Dans l'appareil décrit à l'exemple 1, on charge 0,3 mole de chlorure d'aluminium que l'on refroidit à 0°C puis successivement, sous agitation, 1,6 mole de chlorure d'acétyle en 5 minutes et 0,1 mole de méthyl-2 butanol-2 en 30 minutes. Lorsque l'addition est achevée on laisse remonter la température à 35°C et on maintient 1 heure dans ces conditions.

Le mélange réactionnel résultant de l'acylation est traité par 500 ml d'ammoniac liquide de la manière décrite à l'exemple 1. La masse résultant de ce traitement fait l'objet des opérations décrites à l'exemple 1. Au cours de ce traitement on a isolé un mélange dans lequel on a dosé par RMN :

a) 4 % en mole de pentaméthyl-1,3,4,6,8 naphthyridine-2,7
b) 21 % en mole de diméthyl-2,6 éthyl-4 pyridine
c) 20 % en mole de (diéthyl-2,6 pyridyl-4)-3 butanone-2
d) 46 % en mole de (triméthyl-2,3,6 pyridyl-4)acétone
e) 9 % en mole de tétraméthyl-2,3,4,6 pyridine.

Les composés identifiés présentent des spectres RMN et de masse ayant les caractéristiques suivantes :

a) pentaméthyl-1,3,4,6,8 naphthyridine-2,7 :

- RMN à 250 MHz dans $CDCl_3$
  . singulet (1 H) à 6,92 ppm
- SM à 80 eV : m/e 201 (16,9) ; 200 (100) ; 199 (38) ; 185 (7,1) ; 158 (15,6) ; 144 (5) ; 115 (10,2) ; 51 (4,1) ; 42 (6,4) ;

b) diméthyl-2,6 éthyl-4 pyridine :

- RMN à 250 MHz dans $CDCl_3$
  . triplet (3 H) à 1,22 ppm
  . singulet (6 H) à 2,40 ppm
  . quartet (2 H) à 2,46 ppm
  . singulet (2 H) à 6,66 ppm

- SM à 80 eV : m/e 136 (11,6) ; 135 (100) ; 120 (31,7) ; 107 (7,5) ; 91 (14,6) ; 79 (20,7) ; 77 (14,3) ; 39 (22,1);

c) (diéthyl-2,6 pyridinyl-4)-3 butanone-2 :

- RMN à 250 MHz dans $CDCl_3$
  . doublet (3 H) à 1,48 ppm
  . singulet (3 H) à 2,08 ppm
  . singulet (6 H) à 2,52 ppm
  . quartet (1 H) à 3,66 ppm
  . singulet (2 H) à 6,84 ppm
- SM à 80 eV : m/e 178 (2) ; 177 (13,9) ; 135 (63,4) ; 134 (100) ; 107 (15,5) ; 91 (8,2) ; 77 (7) ; 43 (64,2) ; 39 (12,7) ;

d) (triméthyl-2,3,6 pyridinyl-4)-acétone

- RMN à 250 MHz dans $CDCl_3$
  . singulet (3 H) à 2,12 ppm
  . singulet (3 H) à 2,17 ppm
  . singulet (3 H) à 2,45 ppm
  . singulet (3 H) à 2,49 ppm
  . singulet (2 H) à 3,68 ppm
  . singulet (1 H) à 6,76 ppm
- SM à 80 eV : m/e 178 (3,9) ; 177 (28,6) ; 135 (100) ; 134 (20,8) ; 121 (3) ; 120 (10,4) ; 106 (2,4) ; 93 (8,9) ; 91 (2,5) ; 79 (6,8) ; 77 (12,5) ; 43 (46,7) ; 39 (12,5) ;

e) tétraméthyl-2,3,4,6 pyridine

- RMN à 250 MHz dans $CDCl_3$
  . singulet (3 H) à 2,1 ppm
  . singulet (3 H) à 2,16 ppm
  . singulet (3 H) à 2,32 ppm
  . singulet (3 H) à 2,37 ppm

. singulet (1 H) à 6,74 ppm

- SM à 80 eV : m/e 136 (11,7) ; 135 (100) ; 134 (64,6) ; 120 (25,5) ; 107 (3,5) ; 91 (8,6) ; 79 (15,2) ; 77 (9,6) ; 39 (15) ;

EXEMPLE 7

Dans l'appareil décrit à l'exemple 1, on charge 0,3 mole de chlorure d'aluminium que l'on refroidit à 0°C puis on ajoute, sous agitation, 1 mole de chlorure de propionyle en 3 minutes puis 0,1 mole de diacétone-alcool en 30 minutes. On constate un dégagement d'acide chlorhydrique. Lorsque l'addition est achevée on laisse remonter la température à 35°C et maintient 1 heure dans ces conditions.

Le mélange réactionnel résultant de l'acylation est traité par 400 ml d'ammoniac liquide de la manière décrite à l'exemple 1. La masse résultant de ce traitement fait l'objet des opérations décrites à l'exemple 3. Au cours de ce traitement on a recueilli 13,4 g d'un mélange dans lequel on a dosé par RMN :

a) 44 % en mole de méthyl-3 triéthyl-1,6,8 naphthyridine
b) 15 % en mole de tétraéthyl-1,3,6,8 naphthyridine
c) 3 % en mole de triéthyl-3,6,8 méthyl-1 naphthyridine
d) 30 % en mole de diméthyl-2,4 éthyl-6 pyridine
e) 8 % en mole de diéthyl-2,6 méthyl-4 pyridine,

ce qui correspond respectivement à 30 %, 10 %, 2 %, 21 % et 5,4 % du diacétone-alcool engagé à l'étape d'acylation.

Les composés obtenus présentent des spectres RMN et de masse ayant les mêmes caractéristiques que celles données à l'exemple 3, composés a) et b) et à l'exemple 5 composés a), c) et d).

EXEMPLE 8

Dans l'appareil décrit à l'exemple 1, on charge 0,3 mole de tétrachlorure de titane que l'on refroidit à 0°C puis on ajoute, sous agitation, 1 mole de chlorure de propionyle en 31 minutes puis 0,1 mole de diacétone-alcool en 30 minutes. On constate un dégagement d'acide chlorhydrique. Lorsque l'addition est achevée on laisse remonter la température à 35°C et maintient 2 heures dans ces conditions.

Le mélange réactionnel résultant de l'acylation est traité par

400 ml d'ammoniac liquide de la manière décrite à l'exemple 1. La masse résultant de ce traitement fait l'objet des opérations décrites à l'exemple 3.

Au cours de ce traitement on a recueilli ainsi 15,5 g d'un mélange comprenant en % molaire :

- 10 % de méthyl-3 triéthyl-1,6,8 naphthyridine-2,7
- 1 % de tétraéthyl-1,3,6,8 naphthyridine-2,7
- 1 % de triéthyl-3,6,8 méthyl-1 naphthyridine-2,7
- 74 % de diméthyl-2,4 éthyl-6 pyridine
- 14 % de diéthyl-2,6 méthyl-4 pyridine,

ce qui correspond respectivement à 10 %, 1 %, 1 %, 70 % et 13 % du diacétone-alcool engagé à l'étape d'acylation.

Les produits obtenus en fin de traitement présentent les mêmes caractéristiques spectrales que celles des produits a) et b) de l'exemple 3 et des produits a), c) et d) de l'exemple 5.

EXEMPLE 9

Dans l'appareil décrit à l'exemple 1, on charge 0,15 mole de chlorure d'aluminium que l'on refroidit à 0°C puis on ajoute, sous agitation, 0,8 mole de chlorure d'acétyle en 1 minute puis 0,05 mole de t-butanol en 15 minutes. On constate un dégagement d'acide chlorhydrique. Lorsque l'addition est achevée on laisse remonter la température à 35°C et maintient 30 minutes dans ces conditions.

La masse réactionnelle d'acylation est traitée de la manière suivante : dans 450 ml de chloroforme refroidi à - 60°C on fait arriver bulle à bulle un courant d'ammoniac gazeux (2 bulles/secondes) tandis qu'on ajoute le mélange d'acylation préalablement refroidi à 0°C.

Le mélange réactionnel résultant est ensuite traité de la manière décrite dans l'exemple 1.

On recueille ainsi 6,5 g d'un mélange comprenant 61 % en mole de tétraméthyl-1,3,6,8 naphthyridine-2,7 et 39 % en mole de triméthyl-2,4,6 pyridine ce qui correspond respectivement à 49 % et 32 % du t-butanol engagé à l'étape d'acylation.

Les produits obtenus en fin de traitement présentent les mêmes caractéristiques spectrales que celles des produits de l'exemple 1.

EXEMPLES 10 à 12

La première étape d'acylation est conduite de façon identique à l'exemple 1. Le mélange réactionnel d'acylation est ensuite traité par des solutions saturées d'acétate d'ammonium dans divers solvants. On fait appel à des solutions contenant 1 mole de sel dans 250 ml de solvant.

Les traitements se font à température ambiante. Les masses réactionnelles sont ensuite traitées comme à l'exemple 1 et les composants des mélanges obtenus au cours de ces traitements sont identifiés et dosés par chromatographie en phase gazeuse.

On a obtenu les résultats consignés dans le tableau suivant :

| EXEMPLES | TRAITEMENT | % massique | | |
|---|---|---|---|---|
| | | TMP (1) | DMPA (2) | TMN (3) |
| 10 | $CH_3COONH_4$ / $CH_3OH$ | 32 % | 7 % | 61 % |
| 11 | $CH_3COONH_4$ / $CH_3NO_2$ | 41 % | 4 % | 55 % |
| 12 | $CH_3COONH_4$ / $CH_3CN$ | 29 % | 7,5% | 63,5 % |

(1) triméthyl-2,4,6 pyridine

(2) (diméthyl-2,6 pyridyl-4)acétone

(3) tétraméthyl-1,3,6,8 naphthyridine-2,7

Le composé 2 présente les caractéristiques spectrales suivantes :

. (Diméthyl-2,6 pyridyl-4)acétone :

- RMN à 60 MHZ dans le chloroforme deutéré :
  . singulet (3 H) à 2 ppm
  . singulet (6 H) à 2,38 ppm
  . singulet (2 H) à 3,64 ppm
  . singulet (2 H) à 6,81 ppm
- SM à 80 ev : m/e 163 (20) ; 121 (100) ; 106(4) ; 77 (16) ; 51 (5) ; 43 (62) ; 39 (6,7)

EXEMPLES 13 à 18

On conduit la préparation de la tétraméthyl-1,3,6,8 naphthyridine 2,7 de manière identique à celle décrite à l'exemple 1 tant pour l'étape d'acylation que pour le traitement par $NH_3$ liquide du mélange d'acylation mais en remplaçant à la première étape 0,15 mole de chlorure d'aluminium par 0,15 mole de divers acides de Lewis. Après le traitement de la masse réactionnelle résultant de la deuxième étape selon la méthode décrite à l'exemple 1, on a obtenu les résultats consignés dans le tableau suivant :

| EXEMPLES | ACIDE DE LEWIS | Rendements/au t-butanol engagé % | |
|---|---|---|---|
| | | TMP (1) | TMN (2) |
| 13 (*) | $AlBr_3$ | 34 | 58 |
| 14 | $TiCl_4$ | 61 | 33 |
| 15 | $SnCl_4$ | 70 | 17 |
| 16 | $ZnCl_2$ | 55 | 4 |
| 17 | $SbCl_5$ | 4 | 2 |
| 18 | $FeCl_3$ | 18,5 | 7,5 |

(*) Le mélange réactionnel d'acylation a été maintenu à 35°C pendant 1 h 30 mn.

(1) triméthyl-2,4,6 pyridine

(2) tétraméthyl-1,3,6,8 naphthyridine-2,7

EXEMPLE 19

Dans l'appareil décrit à l'exemple 1, on charge 0,15 mole de $FeCl_3$ et 100 ml de $CHCl_3$ que l'on refroidit à 0°C puis on ajoute, sous agitation, 1 mole de chlorure d'acétyle en 1 minute puis 0,05 mole de t-butanol en 15 minutes. On constate un dégagement d'acide chlorhydrique. Lorsque l'addition est achevée on laisse remonter la température à 35°C et on maintient 3 h 30 mn dans ces conditions.

Le mélange réactionnel résultant de l'acylation est traité par 400 ml d'ammoniac liquide de la manière décrite à l'exemple 1. Le mélange obtenu fait l'objet des opérations décrites dans l'exemple 1.

On recueille ainsi 4 g d'un mélange comprenant 40 % en moles de tétraméthyl-1,3,6,8 naphthyridine-2,7 et 60 % en mole de triméthyl-2,4,6 pyridine, ce qui correspond respectivement à 21 % et 33 % du t-butanol engagé à l'étape d'acylation.

EXEMPLE 20

On conduit la préparation de la tétraméthyl-1,3,6,8 naphthyridine-2,7 de manière identique à celle décrite à l'exemple 1 tant pour l'étape d'acylation que pour le traitement par $NH_3$ liquide du mélange d'acylation mais en changeant la température d'apport des réactifs : 35°C au lieu de 0°C.

Après le traitement de la masse réactionnelle résultant de la deuxième étape selon la méthode décrite à l'exemple 1, on recueille 14,7 g d'un mélange comprenant 63 % en mole de tétraméthyl-1,3,6,8 naphthyridine-2,7 et 34 % en mole de triméthyl-2,4,6 pyridine, ce qui correspond respectivement à 57 % et 34 % du t-butanol engagé à l'étape d'acylation.

EXEMPLE 21

On conduit la préparation de la tétraméthyl-1,3,6,8 naphthyridine-2,7 de manière identique à celle décrite à l'exemple 1 tant pour l'étape d'acylation que pour le traitement par $NH_3$ liquide du

mélange d'acylation en remplaçant 0,1 mole de t-butanol par 0,05 mole de chlorure de t-butyle et en élevant la température de la phase d'acylation à 50°C. La masse réactionnelle d'acylation est maintenue 1 heure à cette température après la fin de l'addition du chlorure d'acétyle.

Après le traitement de la masse réactionnelle résultant de la deuxième étape selon la méthode décrite à l'exemple 1, on recueille un mélange dans lequel on dose 1,81 g de triméthyl-2,4,6 pyridine et 4,65 g de tétraméthyl-1,3?6,8 naphthyridine-2,7, ce qui correspond respectivement à 30 et 50 % du chlorure de t-butyle engagé.

EXEMPLES 22 à 24

On conduit la préparation de la tétraméthyl-1,3,6,8 naphthyrdine-2,7 en opérant selon le mode opératoire de l'exemple 1 mais en présence de 100 ml de divers solvants. Les durées de réaction ont été de 50 minutes à l'exemple 22, 1 h 45 mn à l'exemple 23 et 30 minutes à l'exemple 24.

On a obtenu les résulats consignés ci-après :

| EXEMPLES | SOLVANT | (1) TMP/t-butanol chargé % | (2)TMN/t-butanol chargé % |
|---|---|---|---|
| 22 | nitrométhane | 18 | 53 |
| 23 | nitrobenzène | 41 | 55 |
| 24 | chloroforme | 24,3 | 54,2 |

(1) TMP = triméthyl-2,4,6 pyridine

(2) TMN = tétraméthyl-1,3,6,8 naphthyridine.

EXEMPLE 25

On opère comme à l'exemple 1 en remplaçant à la phase d'acylation le chlorure d'acétyle par le chlorure de butyryle. On porte le contenu du réacteur 45 mn à 35°C. On traite le mélange d'acylation par l'ammoniac liquide comme à l'exemple 1. La masse réactionnelle résultant de cette phase est hydrolysée et l'hydrolysat est extrait au chloroforme comme à l'exemple 1.

La phase chloroformique est évaporée à sec et le résidu est repris par 80 ml de benzène. La n-butyramide est insoluble dans le benzène. On filtre le précipité puis le lave avec 30 ml de benzène. Le filtrat est recueilli et le benzène éliminé par évaporation. Le résidu organique est repris par 200 ml d'une solution aqueuse de soude à 10 % en poids. Cette phase aqueuse est chauffée 3 h à 80°C pour saponifier la n-butyramide restante, puis refroidie à 20°C et extraite par trois fois 30 ml de chloroforme.

Le nouvel extrait chloroformique est évaporé à sec et on recueille 21 g d'un résidu dans lequel on a dosé comme à l'exemple 1 :

- 76 % en mole de tétra-n-propyl-1,3,6,8 naphthyridine-2,7,
- 24 % en mole de di-n-propyl-2,6 méthyl-4 pyridine,

ce qui représente respectivement 59 % et 19 % du t-butanol engagé à l'étape d'acylation.

Les composés obtenus possèdent des spectres RMN et de masse ayant les caractéristiques suivantes :

a) tétra-n-propyl-1,3,6,8 naphthyridine-2,7 :

- RMN à 250 M Hz dans le chloroforme deutéré :
  - . triplet (6 H) à 0,99 ppm
  - . triplet (6 H) à 1,07 ppm
  - . massif (8 H) à 1,83 ppm
  - . triplet (4 H) à 2,83 ppm
  - . triplet (4 H) à 3,32 ppm
  - . singulet (2 H) à 7,16 ppm

- SM à 80 eV : m/e 299 (3,1) ; 298 (9,4) ; 297 (3,5) ; 283 (19,5) ; 270 (22,9) ; 269 (13,3) ; 256 (23,4) ; 255 (100) ; 241 (8,8) ; 225 (6,8) ; 211 (4,2) ; 41 (4,8) ; 27 (4,2).

b) di-n-propyl-2,6 méthyl-4 pyridine :

- RMN à 250 M Hz dans le chloroforme deutéré :
  . triplet (6 H) à 0,90 ppm
  . sextuplet (4 H) à 1,70 ppm
  . singulet (3 H) à 2,26 ppm
  . triplet (4 H) à 2,68 ppm
  . singulet (2 H) à 6,76 ppm
- SM à 80 eV : m/e 177 (2,1) ; 176 (9,3) ; 162 (25) ; 150 (10,7) ; 149 (100) ; 146 (4) ; 133 (9,2) ; 121 (11,7) ; 120 (15) ; 91 (7,1) ; 77 (12,2) ; 41 (15,1) ; 39 (16,4) ; 27 (14,6).

EXEMPLE 26

La première étape d'acylation du t-butanol est conduite comme à l'exemple 1 en remplaçant le chlorure d'acétyle par le chlorure de valéryle. On porte le mélange réactionnel 45 mn à 35°C. La masse réactionnelle d'acylation est traitée par l'ammoniac liquide de la façon décrite à l'exemple 1. Le mélange réactionnel est hydrolysé et l'hydrolysat est extrait au chloroforme comme à l'exemple 1.

La phase chloroformique est évaporée à sec et le résidu organique est repris par de l'hexane (100 ml). La n-valéramide est insoluble dans l'hexane. On filtre le précipité. On le lave avec 30 ml d'hexane. Le filtrat est recueilli et on évapore à sec. Le résidu organique est repris par 200 ml d'une solution aqueuse de soude à 10 % en poids. Cette phase aqueuse est chauffée 2 h à 100°C pour saponifier l'amide restante, puis refroidie à 20°C et extraite par trois fois 30 ml de chloroforme.

Le nouvel extrait chloroformique est évaporé à sec et on recueille 11 g d'un résidu dans lequel on a dosé comme à l'exemple 1 :
- 69 % en mole de tétra-n-butyl-1,3,6,8 naphthyridine-2,7
- 31 % en mole de di-n-butyl-2,6 méthyl-4 pyridine,

ce qui représente respectivement 26 % et 12 % du t-butanol engagé à l'étape d'acylation.

Les composés obtenus possèdent des spectres RMN et de masse ayant les caractéristiques suivantes :

a) tétra-n-butyl-1,3,6,8 naphthyridine-2,7 :

- RMN à 250 MHz dans le chloroforme deutéré :
  . triplet (6 H) à 0,96 ppm
  . triplet (6 H) à 0,98 ppm
  . sextuplet (4 H) à 1,41 ppm
  . sextuplet (4 H) à 1,48 ppm
  . massif (8 H) à 1,76 ppm
  . triplet (4 H) à 2,84 ppm
  . triplet (4 H) à 3,35 ppm
  . singulet (2 H) à 7,14 ppm
- SM à 80 ev : m/e 354 (6,4) ; 325 (21,7) ; 313 (18,1) ; 312 (62) ; 298 (29) ; 297 (100) ; 283 (36,3) ; 270 (10,6) ; 228 (7,9) ; 41 (14,9) ; 29 (13,4) ; 27 (11,7).

b) di-n-butyl-2,6 méthyl-4 pyridine :

- RMN à 250 MHz dans le chloroforme deutéré :
  . triplet (6 H) à 0,94 ppm
  . sextuplet (4 H) à 1,38 ppm
  . quadruplet (4 H) à 1,66 ppm
  . singulet (3 H) à 2,27 ppm
  . triplet (4 H) à 2,71 ppm
  . singulet (2 H) à 6,76 ppm.

<u>EXEMPLE 27</u>

On opère dans les conditions décrites à l'exemple 1 en remplaçant à l'étape d'acylation le t-butanol par l'isobutène. L'apport de l'isobutène se fait de la façon suivante :

Dans un piège gradué refroidi par de la carboglace, on

introduit 9,45 ml d'isobutène (0,1 mole). Ce piège est relié au réacteur par un tuyau en caoutchouc terminé par un embout en verre fritté plongeant dans le liquide présent dans le réacteur. On retire la carboglace et au fur et à mesure que le piège revient à température ambiante, l'isobutène passe dans le réacteur. Cet ajout dure 43 minutes.

Le traitement du mélange d'acylation par l'ammoniac puis de la masse réactionnelle de la deuxième étape sont réalisés comme décrit à l'exemple 1. On a recueilli de cette façon 8,5 g d'un mélange contenant 61 % en mole de tétraméthyl-1,3,6,8 naphthyridine-2,7 et 39 % en mole de triméthyl-2,4,6 pyridine, ce qui représente respectivement 32 % et 21 % de l'isobutène engagé à l'étape d'acylation.

EXEMPLE 28

On opère dans les conditions décrites à l'exemple 1 en remplaçant à l'étape d'acylation le t-butanol par un mélange de 80 % en poids de triméthyl-2,4,4 pentène-1 et de 20 % en poids de triméthyl-2,4,4 pentène-2. L'ajout se fait en 38 minutes. On porte, dès la fin de l'addition du mélange d'oléfines, le contenu du réacteur à 35°C et on maintient 45 minutes dans ces conditions.

Le traitement du mélange d'acylation par l'ammoniac puis de la masse réactionnelle de la deuxième étape sont réalisés comme décrit à l'exemple 1. On a recueilli de cette façon 18 g d'un mélange contenant 65 % de tétraméthyl-1,3,6,8 naphthyridine-2,7, 20 % de triméthyl-2,4,6 pyridine et 15 % de diméthyl-2,6 néopentyl-4 pyridine, ce qui représente respectivement 40,3 %, 12,4 % et 9,3 % du diisobutylène engagé à l'étape d'acylation.

- Caractéristiques de la diméthyl-2,6 néopentyl-4 pyridine

- RMN à 60 MHz dans le chloroforme deutéré
  . singulet (9 H) à 0,9 ppm
  . singulet (2 H) à 2,35 ppm
  . singulet (6 H) à 2,42 ppm
  . singulet (2 H) à 6,80 ppm
- SM à 80 eV : m/e 177 (8) ; 162 (11) ; 121 (100) ; 120 (23) ; 106 (6) ; 71 (6) ; 57 (78).

EXEMPLE 29

On opère dans les conditions décrites à l'exemple 1 en remplaçant à l'étape d'acylation le t-butanol par l'isooctane. On porte, dès la fin de l'addition de l'isooctane, le contenu du réacteur à 35°C et on maintient 3 h 20 mn dans ces conditions.

Le traitement du mélange d'acylation par l'ammoniac puis de la masse réactionnelle de la deuxième étape sont réalisés comme décrit à l'exemple 1. On a recueilli de cette façon 6 g d'un mélange contenant 65 % en mole de tétraméthyl-1,3,6,8 naphthyridine-2,7 et 35 % de triméthyl-2,4,6 pyridine ce qui représente respectivement 24 % et 13 % de l'isooctane engagé à l'étape d'acylation.

EXEMPLE 30

On opère dans les conditions décrites à l'exemple 1 en remplaçant à l'étape d'acylation le t-butanol par l'oxyde de mésityle. L'apport de l'oxyde de mésityle se fait à 35°C. Dès la fin de l'addition de l'oxyde de mésityle, le contenu du réacteur est maintenu 1 h 30 mn dans ces conditions.

Le traitement du mélange d'acylation par l'ammoniac puis de la masse réactionnelle de la deuxième étape sont réalisés comme décrit à l'exemple 1. On a recueilli de cette façon 6,5 g d'un mélange contenant 56 % en mole de tétraméthyl-1,3,6,8 naphthyridine-2,7 et 44 % en mole de triméthyl-2,4,6 pyridine ce qui représente respectivement 23 % et 18 % de l'oxyde de mésityle engagé à l'étape d'acylation.

EXEMPLE 31

Dans l'appareil décrit à l'exemple 1, on charge 0,3 mole de chlorure d'aluminium que l'on refroidit à 0°C puis on ajoute, sous agitation, 1,6 mole de chlorure d'acétyle en 2 minutes. On porte le contenu du réacteur à 35°C. On ajoute 0,1 mole de méthyl-5 hexène-4 one-3 en 30 minutes. On constate un dégagement d'acide chlorhydrique. Lorsque l'addition est achevée, on maintient 1 h 30 mn dans ces conditions.

Le mélange réactionnel résultant de l'acylation est traité par 500 ml d'ammoniac liquide de la manière décrite à l'exemple 1. La masse résultant de ce traitement fait l'objet des opérations décrites à

l'exemple 1.

Le résidu obtenu à l'issue de ce traitement est repris par 30 ml de pentane pour séparer des polymères insolubles par filtration. Le filtrat est recueilli et le pentane évaporé. Le résidu ainsi obtenu est traité par 100 mol d'acide chlorhydrique à 10 % en poids. La phase aqueuse acide est lavée au chloroforme (deux fois 40 ml) puis amenée à pH supérieur à 7 par addition d'une solution aqueuse de soude. La solution aqueuse alcaline est extraite au chloroforme. L'extrait chloroformique est évaporé à sec.

On a recueilli au cours de ce traitement 2 g d'un mélange dans lequel on a dosé par RMN :

a) 30 % en mole d'éthyl-3 triméthyl-1,6,8 naphthyridine-2,7
b) 10 % en mole d'éthyl-1 triméthyl-3,6,8 naphthyridine-2,7
c) 29 % en mole de tétraméthyl-1,3,6,8 naphthyridine-2,7
d) 29 % en mole de diméthyl-2,4 éthyl-6 pyridine
e) 2 % en mole triméthyl-2,4,6 pyridine

ce qui correspond respectivement à 3,3 %, 1,1 %, 3,2 %, 3,2 %, 0,2 % de la méthyl-5 hexène-4 one-3 engagé à l'étape d'acylation.

Les composés identifiés présentent des spectres RMN et de masse ayant les caractéristiques suivantes :

a) éthyl-3 triméthyl-1,6,8 naphthyridine-2,7 :

- RMN à 250 MHz dans le chloroforme deutéré :
  . triplet (3 H) à 1,36 ppm
  . singulet (3 H) à 2,6 ppm
  . quadruplet (2 H) à 2,9 ppm
  . singulet (6 H) à 3,06 ppm
  . singulet (1 H) à 7,14 ppm
  . singulet (1 H) à 7,16 ppm
- SM à 80 eV : m/e 201 (9,5 ) ; 200 (57,4) ; 199 (100) ; 172 (16,9) ; 171 (3,5) ; 91 (3,9) ; 63 (4,5) ; 51 (5,8) ; 42 (8,2) ; 39 (11,2) ; 27 (4,4).

b) éthyl-1 triméthyl-3,6,8 naphthyridine-2,7 :

- RMN à 250 MHz dans le chloroforme deutéré
  - . triplet (3 H) à 1,4 ppm
  - . singulet (6 H) à 2,6 ppm
  - . singulet (3 H) à 3,06 ppm
  - . quadruplet (2 H) à 3,4 ppm
  - . singuelet (2 H) à 7,14 ppm
- SM à 80 eV : m/e 201 (8,2) ; 200 (49,3) ; 199 (29,8) ; 197 (4) ; 186 (13) ; 185 (100) ; 184 (7,5) ; 183 (7,9) ; 172 (16,1) ; 171 (5,3) ; 99 (6,5) ; 77 (8,4) ; 51 (5,8) ; 42 (6,3) ; 39 (11,6).

EXEMPLE 32

Dans l'appareil décrit à l'exemple 1, on charge 0,45 mole de chlorure d'aluminium que l'on refroidit à 0°C puis on ajoute, sous agitation, 2,4 moles de chlorure d'acétyle en 2 minutes. On porte le contenu du réacteur à 35°C. On ajoute 0,15 mole de diméthyl-2,5 hexène-4 one-3 en 30 minutes. On constate un dégagement d'acide chlorhydrique. Lorsque l'addition est achevée, on maintient 2 heures dans ces conditions.

Le mélange réactionnel résultant de l'acylation est traité par 500 ml d'ammoniac liquide de la manière décrite à l'exemple 1. La masse résultant de ce traitement fait l'objet des opérations décrites à l'exemple 1.

Le résidu obtenu à l'issue de ce traitement est repris par 30 ml de pentane pour séparer des polymères insolubles par filtration. Le filtrat est recueilli et le pentane évaporé. Le résidu ainsi obtenu est traité par de la soude concentrée à reflux pendant 3 heures. On extrait ensuite la masse réactionnelle par du chloroforme puis évapore ce dernier de l'extrait chloroformique.

On a recueilli au cours de ce traitement 2 g d'un mélange dans lequel on a dosé par RMN :

a) 30 % en mole d'isopropyl-3 triméthyl-1,6,8 naphtyridine-2,7

b) 26 % en mole de tétraméthyl-1,3,6,8 naphthyridine-2,7

c) 33 % en mole de diméthyl-2,4 isopropyl-6 pyridine

d) 11 % en mole de triméthyl-2,4,6, pyridine,

ce qui correspond respectivement à 2,4 %, 1,9 %, 2,5 %, 0,8 % de la diméthyl-2,5 hexène-4 one-3 engagé à l'étape d'acylation.

Les composés identifiés présentent des spectres RMN et de masse ayant les caractéristiques suivantes :

a) isopropyl-3 triméthyl-1,6,8 naphthyridine-2,7 :

- RMN à 250 MHz dans le chloroforme deutéré :
  . doublet (6 H) à 1,34 ppm
  . singulet (3 H) à 2,48 ppm
  . septuplet (1 H) à 3,06 ppm
  . singulet (6 H) à 3,08 ppm
  . singulet (1 H) à 7,16 ppm
  . singulet (1 H) à 7,20 ppm
- SM à 80 eV : m/e 215 (7,6) ; 214 (45,7) ; 213 (42,7) ; 200 (16,6) ; 199 (100) ; 186 (51,8) ; 172 (10,3) ; 93 (14,1) ; 42 (11) ; 41 (8,1) ; 39 (14,3).

b) diméthyl-2,4 isopropyl-6 pyridine :

- RMN à 250 MHz dans le chloroforme deutéré
  . doublet (6 H) à 1,28 ppm
  . singulet (3 H) à 2,28 ppm
  . singulet (3 H) à 2,48 ppm
  . septuplet (1 H) à 3 ppm
  . singulet (2 H) à 6,8 ppm
- SM à 80 eV : m/e 150 (1,8) ; 149 (18,1) ; 148 (23,8) ; 134 (100) ; 121 (29,3) ; 107 (21,8) ; 106 (10,7) ; 91 (5,2) ; 77 (12,7) ; 41 (16,5) ; 39 (24,3) ; 27 (12,7).

REVENDICATIONS

1°) Nouvelles naphthyridines-2,7 caractérisées en ce qu'elles répondent à la formule générale :

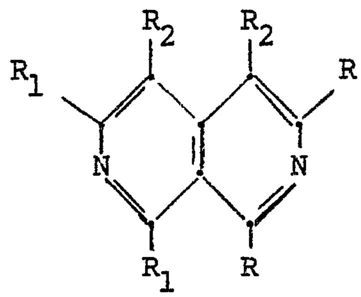

(I)

dans laquelle :

- les symboles $R_1$, qui sont identiques, représentent des radicaux alkyles linéaires ou ramifiés, ne comportant pas dans ce dernier cas d'atome de carbone tertiaire juxtanucléaire, ayant de 1 à 20 atomes de carbone, éventuellement substitués par un ou plusieurs radicaux aromatiques ou hétérocycliques ou par un ou plusieurs groupes fonctionnels situés au moins en position $\gamma$ par rapport au carbone cyclique du cycle naphthyridine,
- l'un quelconque au moins des symboles R représente un radical $R_1$ tel que défini ci-avant et, le cas échéant, l'autre symbole R représente un radical $R_3$ défini comme un radical alkyle linéaire ou ramifié ayant de 1 à 20 atomes de carbone, éventuellement substitués par un ou plusieurs radicaux aromatiques ou hétérocycliques ou par un ou plusieurs groupes fonctionnels situés au moins en position $\gamma$ par rapport au carbone cyclique du cycle naphthyridine, et qui peut être identique ou différent des radicaux $R_1$.
- les symboles $R_2$ représentent un atome d'hydrogène ou un radical méthyle avec la restriction que l'un quelconque au moins des symboles $R_2$ représente un atome d'hydrogène.

2°) Nouvelles naphthyridines-2,7 selon la revendication 1, caractérisée en ce que dans la formule (I) les radicaux $R_1$ représentent un groupe alkyle ayant de 1 à 5 atomes de carbone, $R_2$ un atome d'hydrogène et, quand l'un des symboles R est un radical $R_3$, ce dernier

représente un radical alkyle ayant de 1 à 5 atomes de carbone identique ou différent de $R_1$.

3°) Nouvelles naphthyridines-2,7 selon la revendication 1, caractérisée en ce qu'il s'agit de la tétraméthyl-1,3,6,8 naphthyridine-2,7 ; de la tétraéthyl-1,3,6,8 naphthyridine-2,7 ; de la tétraisopropyl-1,3,6,8 naphthyridine-2,7 ; de la méthyl-3 triéthyl-1,6,8 naphthyridine-2,7 ; de la méthyl-1 triéthyl-3,6,8 naphthyridine-2,7 ; de la pentaméthyl-1,3,4,6,8 naphthyridine-2,7 ; de la tétra-n-butyl-1,3,6,8 naphthyridine-2,7 ; de la tétra-n-propyl-1,3,6,8 naphthyridine-2,7 ; de l'éthyl-3 triméthyl-1,6,8 naphthyridine-2,7 ; de l'éthyl-1 triméthyl-3,6,8 naphthyridine-2,7 ; de l'isopropyl-3 triméthyl-1,6,8 naphthyridine-2,7.

4°) Procédé de préparation des polyalkylnaphthyridines-2,7 de formule (I), caractérisé en ce qu'il comprend :

A - une première étape au cours de laquelle on fait réagir en milieu anhydre, en présence d'un acide de Lewis comme catalyseur, un agent acylant de formule générale :

$$R_1 - \overset{\overset{O}{\|}}{C} - Z \qquad (II)$$

dans laquelle :

. $R_1$ a la définition donnée ci-avant,

. Z représente un atome d'halogène

avec un composé éthylénique de formule générale :

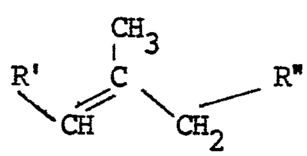

(III)

dans laquelle l'un quelconque des radicaux R' et R" représente un atome d'hydrogène ou le radical méthyle et l'autre un atome d'hydrogène ou un groupe alkylcarbonyle $R_3$ - CO - où $R_3$ a la signification donnée à la revendication 1, ou avec un composé susceptible de conduire à la formation "in situ" d'un composé éthylénique de formule (III) dans les conditions de la réaction et répondant à la formule générale :

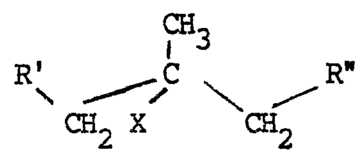

(IV)

dans laquelle :

. R' et R" ont la signification donnée ci-avant ;

. X représente : un atome d'hydrogène ; un atome d'halogène ; un reste de formule $R_6O$- où $R_6$ est un atome d'hydrogène ou un radical méthyle ou éthyle ; un reste ester Q - O - où Q représente le reste d'un acide minéral ou carboxylique ou sulfonique ; un radical isobutyle ou isobutènyle avec la restriction que R' et R" représentent dans ce cas un atome d'hydrogène.

B - une seconde étape au cours de laquelle la masse réactionnelle provenant de la première étape est traitée, en absence d'eau, par de l'ammoniac anhydre ou un sel d'ammonium, éventuellement après élimination de l'agent acylant et/ou du composé éthylénique ou de son précurseur en excès.

5°) Procédé selon la revendication 4, caractérisé en ce que l'on utilise comme composé éthylénique des composés de formule (III) dans laquelle l'un des radicaux R' et R" représente un atome d'hydrogène et l'autre un atome d'hydrogène ou le reste acétyle.

6°) Procédé selon la revendication 4, caractérisé en ce que l'on utilise comme précurseur de composés éthyléniques des composés de formule (IV) dans laquelle l'un des radicaux R' et R" représente un atome d'hydrogène et l'autre un atome d'hydrogène ou le reste acétyle et X un atome de chlore, de brome, un groupe hydroxyle, un radical méthoxy, un radical méthylcarbonyloxy.

7°) Procédé selon l'une quelconque des revendications 4 à 6, caractérisé en ce que l'on utilise comme composés de formule (III) ou (IV) le chlorure de t-butyle, le t-butanol, le méthyl-2 butanol-2, la méthyl-4 hydroxy-4 pentanone-2, la méthyl-4 méthoxy-4 pentanone-2, l'isobutène, le triméthyl-2,4,4 pentène-1, le triméthyl-2,4,4 pentène-2 et ses mélanges avec le triméthyl-2,4,4 pentène-1, l'oxyde de mésityle, l'isooctane, la méthyl-5 hexène-4 one-3, la diméthyl-2,5 hexène-4 one-3.

8°) Procédé selon l'une quelconque des revendications 4 à 7, caractérisé en ce que l'on utilise comme agent acylant un bromure ou un chlorure d'acyle.

9°) Procédé selon la revendication 8, caractérisé en ce que l'on utilise comme agent acylant les bromures et chlorures d'acétyle, de propionyle, d'isobutyryle, de valéryle et de n-butyryle.

10°) Procédé selon l'une quelconque des revendications 4 à 9, caractérisé en ce que l'on utilise comme acide de Lewis des halogénures de métaux des groupes 3a à 5a et des halogénures des métaux de transitions de la classification périodique des éléments.

11°) Procédé selon la revendication 10, caractérisé en ce que l'on utilise comme acide de Lewis un chlorure ou un bromure de Ti, Fe, Zn, Al, Sn, Sb.

12°) Procédé selon l'une quelconque des revendications 4 à 11, caractérisé en ce que la quantité d'agent acylant utilisée à l'étape d'acylation est d'au moins 3 moles par mole de composé éthylénique de formule (III) ou de précurseur de formule (IV).

13°) Procédé selon la revendication 12, caractérisé en ce que la quantité d'agent acylant est au moins égale à 4 moles et au plus égale à 20 moles par mole de composé éthylénique de formule (III) ou de précurseur de formule (IV).

14°) Procédé selon l'une quelconque des revendications 4 à 13, caractérisé en ce que la quantité d'acide de Lewis est d'au moins 0,5 mole par mole de composé éthylénique de formule (III) ou de précurseur de formule (IV).

15°) Procédé selon la revendication 14, caractérisé en ce que la quantité d'acide de Lewis est d'au moins 2 moles et d'au plus 6 moles par mole de composé éthylénique de formule (III) ou de précurseur de formule (IV).

16°) Procédé selon l'une quelconque des revendications 4 à 15, caractérisé en ce que la quantité d'ammoniac ou de sel d'ammonium utilisés à la seconde phase du procédé est d'au moins 2 moles par mole de composé éthylénique de formule (III) ou de précurseur de formule (IV).

17°) Procédé selon l'une quelconque des revendications 4 à 16, caractérisé en ce que l'ammoniac est utilisé à l'état liquide.

18°) Procédé selon l'une quelconque des revendications 4 à 16, caractérisé en ce que la deuxième phase est réalisée par addition de la masse réactionnelle d'acylation dans une solution d'ammoniac dans un solvant organique inerte.

19°) Procédé selon l'une quelconque des revendications 4 à 16, caractérisé en ce que le sel d'ammonium répond à la formule générale :

$$A^{n-}(NH_4^+)_n \quad \text{(VII)}$$

dans laquelle :

. A représente le reste d'un acide minéral ou organique

. n représente un nombre entier supérieur ou égal à 1.

20°) Procédé selon la revendication 19, caractérisé en ce que le sel d'ammonium est un carbonate, un carboxylate ou un halogénure d'ammonium.

21°) Procédé selon la revendication 20, caractérisé en ce que le sel d'ammonium est choisi dans le groupe formé par $(NH_4)_2CO_3$ ; le formiate, l'acétate, le propionate, le chlorure et le bromure d'ammonium.

22°) Procédé selon l'une quelconque des revendications 4 à 21, caractérisé en ce que la température utilisée à la première phase est comprise entre -10 et 100°C.

23°) Procédé selon l'une quelconque des revendications 4 à 22, caractérisé en ce que la température utilisée à la seconde étape est comprise entre -60°C et + 20°C.

0088707

REVENDICATIONS AUTRICHE

1°) Procédé de préparation des polyalkylnaphthyridines-2,7 de formule générale :

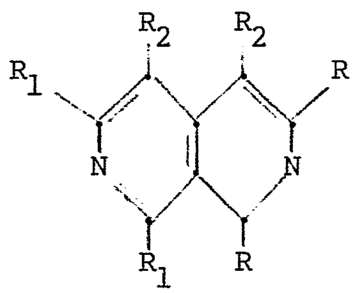

(I)

dans laquelle :

- les symboles $R_1$, qui sont identiques, représentent des radicaux alkyles linéaires ou ramifiés, ne comportant pas dans ce dernier cas d'atome de carbone tertiaire juxtanucléaire, ayant de 1 à 20 atomes de carbone, éventuellement substitués par un ou plusieurs radicaux aromatiques ou hétérocycliques ou par un ou plusieurs groupes fonctionnels situés au moins en position γ par rapport au carbone cyclique du cycle naphthyridine,
- l'un quelconque au moins des symboles R représente un radical $R_1$ tel que défini ci-avant et, le cas échéant, l'autre symbole R représente un radical $R_3$ défini comme un radical alkyle linéaire ou ramifié ayant de 1 à 20 atomes de carbone, éventuellement substitués par un ou plusieurs radicaux aromatiques ou hétérocycliques ou par un ou plusieurs groupes fonctionnels situés au moins en position γ par rapport au carbone cyclique du cycle naphthyridine, et qui peut être identique ou différent des radicaux $R_1$.
- les symboles $R_2$ représentent un atome d'hydrogène ou un radical méthyle avec la restriction que l'un quelconque au moins des symboles $R_2$ représente un atome d'hydrogène.

caractérisé en ce qu'il comprend :

A - une première étape au cours de laquelle on fait réagir en

milieu anhydre, en présence d'un acide de Lewis comme catalyseur, un agent acylant de formule générale :

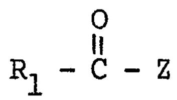 (II)

dans laquelle :

. $R_1$ a la définition donnée ci-avant,

. Z représente un atome d'halogène

avec un composé éthylénique de formule générale :

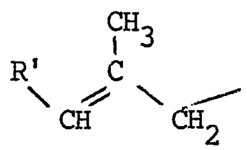 (III)

dans laquelle l'un quelconque des radicaux R' et R" représente un atome d'hydrogène ou le radical méthyle et l'autre un atome d'hydrogène ou un groupe alkylcarbonyle $R_3$ - CO - où $R_3$ a la signification donnée ci-avant, ou avec un composé susceptible de conduire à la formation "in situ" d'un composé éthylénique de formule (III) dans les conditions de la réaction et répondant à la formule générale :

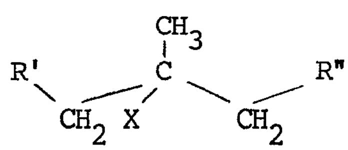 (IV)

dans laquelle :

. R' et R" ont la signification donnée ci-avant ;

. X représente : un atome d'hydrogène ; un atome d'halogène ; un reste de formule $R_6O$- où $R_6$ est un atome d'hydrogène ou un radical méthyle ou éthyle ; un reste ester Q - O - où Q représente le reste d'un acide minéral ou carboxylique ou sulfonique ; un radical isobutyle ou isobutényle avec la restriction que R' et R" représentent dans ce cas un atome d'hydrogène.

B - une seconde étape au cours de laquelle la masse réactionnelle provenant de la première étape est traitée, en absence d'eau, par de l'ammoniac anhydre ou un sel d'ammonium, éventuellement

après élimination de l'agent acylant et/ou du composé éthylénique ou de son précurseur en excès.

2°) Procédé selon la revendication 1, caractérisé en ce que l'on utilise comme composé éthylénique des composés de formule (III) dans laquelle l'un des radicaux R' et R" représente un atome d'hydrogène et l'autre un atome d'hydrogène ou le reste acétyle.

3°) Procédé selon la revendication 1, caractérisé en ce que l'on utilise comme précurseur de composés éthyléniques des composés de formule (IV) dans laquelle l'un des radicaux R' et R" représente un atome d'hydrogène et l'autre un atome d'hydrogène ou le reste acétyle et X un atome de chlore, de brome, un groupe hydroxyle, un radical méthoxy, un radical méthylcarbonyloxy.

4°) Procédé selon l'une quelconque des revendications 1 à 3, caractérisé en ce que l'on utilise comme composés de formule (III) ou (IV) le chlorure de t-butyle, le t-butanol, le méthyl-2 butanol-2, la méthyl-4 hydroxy-4 pentanone-2, la méthyl-4 méthoxy-4 pentanone-2, l'isobutène, le triméthyl-2,4,4 pentène-1, le triméthyl-2,4,4 pentène-2 et ses mélanges avec le triméthyl-2,4,4 pentène-1, l'oxyde de mésityle, l'isooctane, la méthyl-5 hexène-4 one-3, la diméthyl-2,5 hexène-4 one-3.

5°) Procédé selon l'une quelconque des revendications 1 à 4, caractérisé en ce que l'on utilise comme agent acylant un bromure ou un chlorure d'acyle.

6°) Procédé selon la revendication 5, caractérisé en ce que l'on utilise comme agent acylant les bromures et chlorures d'acétyle, de propionyle, d'isobutyryle, de valéryle et de n-butyryle.

7°) Procédé selon l'une quelconque des revendications 1 à 6, caractérisé en ce que l'on utilise comme acide de Lewis des halogénures de métaux des groupes 3a à 5a et des halogénures des métaux de transitions de la classification périodique des éléments.

8°) Procédé selon la revendication 7, caractérisé en ce que l'on utilise comme acide de Lewis un chlorure ou un bromure de Ti, Fe, Zn, Al, Sn, Sb.

9°) Procédé selon l'une quelconque des revendications 1 à 8, caractérisé en ce que la quantité d'agent acylant utilisée à l'étape d'acylation est d'au moins 3 moles par mole de composé éthylénique de

formule (III) ou de précurseur de formule (IV).

10°) Procédé selon la revendication 9, caractérisé en ce que la quantité d'agent acylant est au moins égale à 4 moles et au plus égale à 20 moles par mole de composé éthylénique de formule (III) ou de précurseur de formule (IV).

11°) Procédé selon l'une quelconque des revendications 1 à 10, caractérisé en ce que la quantité d'acide de Lewis est d'au moins 0,5 mole par mole de composé éthylénique de formule (III) ou de précurseur de formule (IV).

12°) Procédé selon la revendication 10, caractérisé en ce que la quantité d'acide de Lewis est d'au moins 2 moles et d'au plus 6 moles par mole de composé éthylénique de formule (III) ou de précurseur de formule (IV).

13°) Procédé selon l'une quelconque des revendications 1 à 12, caractérisé en ce que la quantité d'ammoniac ou de sel d'ammonium utilisés à la seconde phase du procédé est d'au moins 2 moles par mole de composé éthylénique de formule (III) ou de précurseur de formule (IV).

14°) Procédé selon l'une quelconque des revendications 1 à 13, caractérisé en ce que l'ammoniac est utilisé à l'état liquide.

15°) Procédé selon l'une quelconque des revendications 1 à 13, caractérisé en ce que la deuxième phase est réalisée par addition de la masse réactionnelle d'acylation dans une solution d'ammoniac dans un solvant organique inerte.

16°) Procédé selon l'une quelconque des revendications 1 à 13, caractérisé en ce que le sel d'ammonium répond à la formule générale :

$$A^{n-}(NH_4^+)_n \quad \text{(VII)}$$

dans laquelle :

. A représente le reste d'un acide minéral ou organique

. n représente un nombre entier supérieur ou égal à 1.

17°) Procédé selon la revendication 16, caractérisé en ce que le sel d'ammonium est un carbonate, un carboxylate ou un halogénure d'ammonium.

18°) Procédé selon la revendication 17, caractérisé en ce que

le sel d'ammonium est choisi dans le groupe formé par $(NH_4)_2CO_3$ ; le formiate, l'acétate, le propionate, le chlorure et le bromure d'ammonium.

19°) Procédé selon l'une quelconque des revendications 1 à 18, caractérisé en ce que la température utilisée à la première phase est comprise entre -10 et 100°C.

20°) Procédé selon l'une quelconque des revendications 1 à 19, caractérisé en ce que la température utilisée à la seconde étape est comprise entre -60°C et + 20°C.

Office européen des brevets

0088707

Numéro de la demande

# RAPPORT DE RECHERCHE EUROPEENNE

EP 83 42 0031

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| | Néant<br>----- | | C 07 D 471/04 //<br>C 07 D 213/12<br>A 61 K 31/435<br>(C 07 D 471/04<br>C 07 D 221/00<br>C 07 D 221/00 ) |
| | | | **DOMAINES TECHNIQUES RECHERCHES (Int. Cl. 3)** |
| | | | C 07 D 471/00<br>C 07 D 213/00 |

Le présent rapport de recherche a été établi pour toutes les revendications

| Lieu de la recherche | Date d'achèvement de la recherche | Examinateur |
|---|---|---|
| LA HAYE | 25-05-1983 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant